# EUROPEAN PATENT SPECIFICATION

(11) **EP 2 900 333 B1**
(45) Date of publication and mention of the grant of the patent: **02.08.2017**
(21) Application number: 13773536.1
(22) Date of filing: 25.09.2013
(51) Int. Cl.: A61Q 9/02, A61K 8/81, A61K 8/86, A61K 8/90, B26B 21/44, A61K 8/42

(54) **A SKIN ENGAGING MEMBER COMPRISING AT LEAST ONE THERMALLY RESILIENT SENSATE**
HAUTKONTAKTELEMENT MIT WENIGSTENS EINER THERMISCH FLEXIBLEN EMPFINDUNG
ÉLÉMENT POUR CONTACT AVEC LA PEAU, COMPRENANT AU MOINS UN AGENT SENSORIEL THERMIQUEMENT RÉSILIENT

(30) Priority: 28.09.2012 US 201261707376 P
(43) Date of publication of application: 05.08.2015
(73) Proprietor: The Gillette Company LLC, Boston, MA 02127 (US)
(72) Inventor: WANG, Xiandong, Boston, Massachusetts 02127 (US); JABALPURWALA, Fatima, Abdulhussain, Andover, Massachusetts 01810 (US); BAKES, Katharine, Anne, Boston, Massachusetts 02127 (US)
(74) Representative: Kohol, Sonia
(86) International application number: PCT/US2013/061584
(87) International publication number: WO 2014/052390

(56) References cited:
- EP-A1- 2 068 150
- US-A- 5 713 131
- US-A1- 2008 300 314
- US-A1- 2011 081 303

## Description

### BACKGROUND OF THE INVENTION

The use of shaving aids on razor blades to provide lubrication benefits during the shave is known. *See e.g.,* U.S. Patents 7,121,754; 6,298,558; 5,711,076; 5,134,775; 6,301,785 and U.S. Patent Publ. Nos. 2009/0223057, 2006/0225285. The use of certain cooling sensates in shaving aids has also been disclosed. *See e.g.,* U.S. Patent Pubs. 2007/0077331, 2008/031166, 2008/0300314A1; U.S. Patent Nos 5,451,404, and 7,482,373; and WO2007/036814A2. For example, it has been described that cooling agents and/or essential oils can be included in the shaving aid to deliver a fresh and cool feel after contact. It has been reported, however, that a substantial amount of the essential oil can be lost due to volatilization prior to use. *See* U.S. Patent No. 5,095,619. U.S. Patent No. 5,713,131 attempts to fix this potential problem by introducing non-volatile cooling agents into the shave aid, such as non-volatile menthol analogs. Examples of other shave aids containing menthol and other actives are disclosed in U.S. Patents 5,095,619, 6,298,558, 6,944,952, and 6,295,733. *See also,* U.S. Patent Nos. 5,653,971 (disclosing a shaving aid which includes an improved shaving aid composite (or lubricating strip) which contains an inclusion complex of a skin soothing agent, such as menthol, with a cyclodextrin) and, 5,713,131 (disclosing a non-volatile cooling agent, such as Cooling Agent 10, WS-3, WS-23, Frescolat ML, Frescolat MGA and Menglytate). It has been reported that these shaving aids deliver cooling agent during use.

Many ingredients that are normally used in skin care, however, are not easy to use in a conventional extruded shaving aid. This is because many shaving aids are extruded through a die or otherwise processed at high temperatures, such as from about 160°C to about 180°C. Formulating extruded shaving aids with cooling agents is challenging since many of these cooling agents have boiling points below the typical shaving aid extrusion temperature. Furthermore, extrusion subjects the shaving aid compositions to high pressure which can also add to the degradation of the cooling agents. One commonly used cooling agent is L-menthol. The addition of this cooling agent as a neat ingredient in a shaving aid has been described but the cooling affect is believed to be limited by the concentration of L-menthol used and lack of shelf life due to its high volatility. Cooling agents having greater cooling intensity are known but they tend to have even lower evaporating temperatures making them less likely to be suitable for the high temperatures and pressures used in conventional shaving aid extrusion.

Various cooling technologies have also been described in cosmetic and / or oral care formulations. *See e.g.* U.S. Patent Pub. Nos 2009/0311206 and 2009/0306152, both assigned to Beiersdorf, 20060276667, 2010/0086498, 2010/0086498, 2011/0081303, and 2011/0082204. Not all cooling technologies however are suitable for processing in normal shaving aid making conditions. In particular, some cooling technologies are believed to be so volatile that they can be lost during the shaving aid making process or otherwise become less active such that they are not perceivable during use. As such, there is a need for technologies which can survive the skin engaging member making process while maintaining sufficient molecular activity to provide meaningful or long lasting cooling benefit.

### SUMMARY OF THE INVENTION

One aspect of this invention relates to a skin engaging member, for use with a hair removal device, such as a razor comprising a hair removal head, one or more elongated edges positioned on said cartridge; and the skin engaging member positioned on the cartridge 1, said skin engaging member comprising a matrix comprising at least one of: a water soluble polymer selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof an emollient, a soap base, and a mixture thereof; and at least one thermally resilient sensate comprising a menthane carboxylic acid-N-(4-methoxyphenyl)-amide of formula: 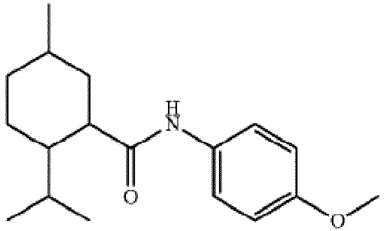 The thermally resilient sensate can be included at various levels, such as from about 0.01% to about 25%, alternatively from about 1% to about 20%, alternatively from about 5% to about 15%, alternatively from about 7% to 13%, alternatively about 10%.

A further aspect of the invention relates to a hair removal device comprising the aforementioned skin engaging member. Another aspect of the invention relates to a method of making a skin engaging member comprising the thermally resilient sensate.

### BRIEF DESCRIPTION OF THE DRAWINGS

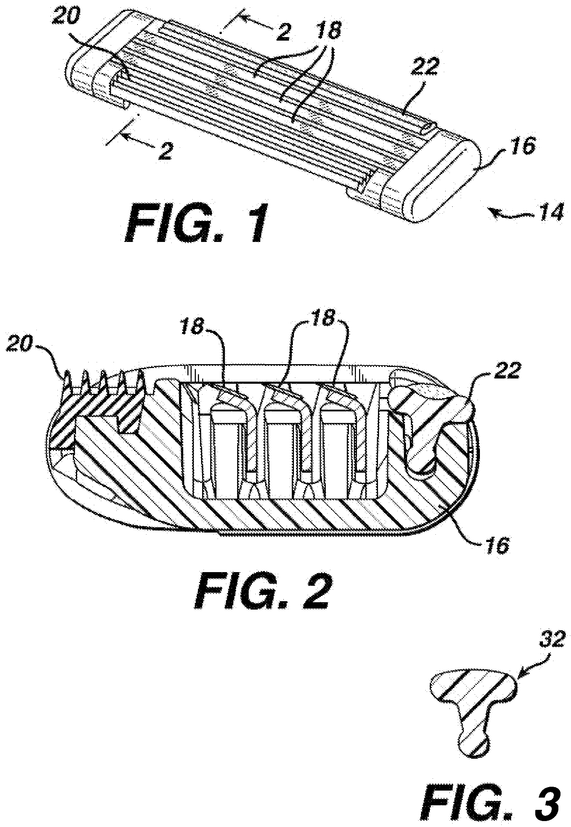

Fig. 1 is a perspective view of a hair removal head (razor cartridge) which includes a skin engaging member of the present invention. Fig. 2 is a sectional view taken along line 2-2 of Fig. 1. Fig. 3 is a side elevation view of a second type of skin engaging member of the present invention.

### DETAILED DESCRIPTION OF THE INVENTION

### I. Thermally Resilient Sensates

It is now well established that sensations such as cool or cold can be attributed to activation of receptors at peripheral nerve fibers by a stimulus such as low temperature or a chemical coolant, which produces electrochemical signals that travel to the brain, which then interprets, organizes and integrates the incoming signal(s) into a perception or sensation. Different classes of receptors have been implicated in sensing cold temperatures or chemical coolant stimuli at mammalian sensory nerve fibers. Among these receptors, a major candidate involved in sensing cold has been identified and designated as cold- and menthol-sensitive receptor (CMR1) or TRPM8. The TRPM8 nomenclature for the receptor comes from its characterization as a non-selective cation channel of the transient receptor potential (TRP) family that is activated by stimuli including low temperatures, menthol and other chemical coolants. However, the precise mechanisms underlying the perception of a pleasant cooling sensation on skin or oral surfaces are presently not clearly understood. While it has been demonstrated that the TRPM8 receptor is activated by menthol and other coolants, it is not fully understood what other receptors may be involved and to what extent these receptors need to be stimulated or perhaps suppressed in order that the overall perceived sensation would be pleasant, cooling and refreshing. Sensates have been described in various applications. *See* e.g. U.S. Patent Publ No. 2010/0086498.

The skin engaging member of the present invention comprises at least one thermally resilient sensate. Thermally resilient sensates are defined herein as sensate ingredients which are capable of surviving conventional shaving aid (skin engaging member) extrusion conditions but still remain sufficiently active to provide cooling or tingling sensations, typically perceptible by the user, on skin during use in a shaving context. Without intending to be bound by theory, it is believed that the thermally resilient sensate of the present invention can deliver greater cooling intensity even after it is extruded into a skin engaging member, compared to sensates that are volatile and can be lost in the making process. In some embodiments, the thermally resilient sensate retains at least 50% of its cooling intensity compared to when it is applied onto skin at the same concentration in a liquid medium, or at least 70%, or at least 90%. Those of skill in the art will understand that skin engaging members may also comprise hair removal or shaving aids and such skin engaging members are also commonly referred to as lubricating strips suitable for use on the skin contacting portions of hair removal devices, especially razor cartridges.

Furthermore, the thermally resilient sensates of the present invention provide a greater cooling intensity when provided in a skin engaging member beyond the cooling intensity of L-menthol, preferably at least 1.5 times greater cooling intensity, more preferably at least 5 times greater cooling intensity, even more preferably at least about 10 times greater cooling intensity, up to about 20 times greater cooling intensity.

Suitable thermally resilient sensates include synthetic derivatives of cyclohexane, specifically menthane carboxylic acid-N-(4-methoxyphenyl)-amide. The at least one sensate can be included at a level of from about 0.01% to about 25%, alternatively from about 1% to about 20%, alternatively from about 5% to about 15%, alternatively from about 7% to 13%, alternatively about 10%. Without intending to be bound by theory, it is believed that these levels of thermally resilient sensate provide for an appreciable performance benefit to a meaningful amount of users, particularly at a level of above 5%, and at a level below 15%. It is believe that although some users may find lower levels enjoyable, many may find that there is too low impact. Similarly, although some users may enjoy a higher level above 15%, it may be too much for the majority of intended consumers.

Without intending to be bound by theory, it is believed that the cooling intensities of these thermally resilient sensates are about 1.5 times the cooling intensity of L-menthol. *See* Leffingwell, John C. PhD, Cool without Menthol & Cooler than Menthol and Cooling Compounds as Insect Repellents (Leffingwell & Associates, Last updated May 4, 2011).

The skin engaging member can also optionally comprise an additional coolant.

### a. Menthane carboxylic acid-N-(4-methoxyphenyl)-amide

The thermally resilient sensate comprises a menthane carboxylic acid-N-(4-methoxyphenyl)-amide having Formula A or, preferably, Formula B, below.

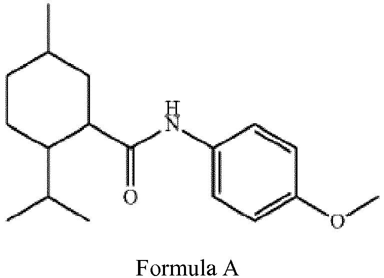

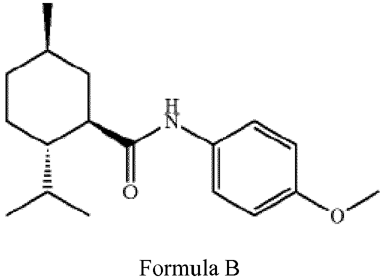

Non-limiting examples of such menthane carboxylic acid-N-(4-methoxyphenyl)-amides are disclosed in U.S. Patent Pub. 2011/0081303, and 2010/0086498. This material is also described under CAS#68489-09-8, may also be named (1*R**,2*S**)*-N-*(4-Methoxyphenyl)-5-methyl-2-(1-methylethyl)cyclohexanecarboxamide
and is commercially available as SC1, WS-12 or Frescolat MMC by Symrise, Inc.

### b. N-substituted menthanecarboxamide

In some preferred embodiments, the thermally resilient sensate also comprises an N-substituted menthanecarboxamide, specifically of the Formula I, below. 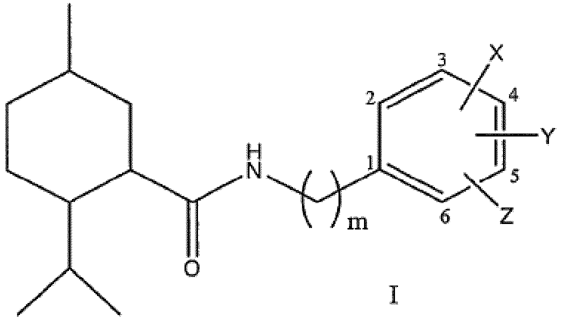 in which m is 0 or 1, Y and Z are selected independently from the group consisting of H, OH, C1-C4 straight or branched alkyl, or, a C1-C4 straight or branched alkoxy, X is (CH2)n-R, where n is 0 or 1 and R is a group with non-bonding electrons, with the provisos that: (a) when Y and Z are H. X is not F, OH, MeO or NO2 in the 4-position and is not OH in the 2 or 6-position (b) when Y or Z is H then X, Y and Z are such that (i) the groups in the 3- and 4-positions are not both OMe, (ii) the groups in the 4- and 5-positions are not both OMe, (iii) the groups in 3- and 5- positions are not OMe if the group in the 4-position is OH, and (iv) the groups in the 3- and 5-positions are not OH if the group in the 4- position is methyl.

The preferred compounds are those in which X is in the 4-position. The most preferred compounds are when X is in the 4-position and Y and Z are H, OH, Me or OMe.

The non-bonding electrons are halogens, OH, OMe, NO2, CN, Ac, SO2NH2, CHO, CO2H and C1-C4 alkyl carboxylates such as CO2Et.

On specific example of a suitable N-substituted menthanecarboxamide is N-[4-(cyanomethyl)phenyl]-(1R,2S,5R)-2-isopropyl-5-methylcyclohexanecarboxamide of Formula II.

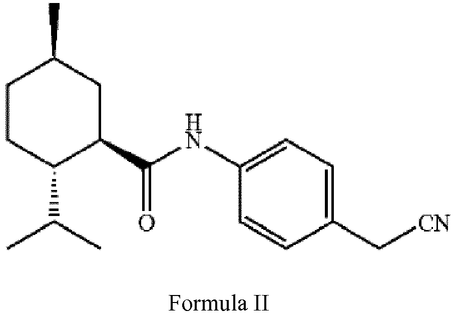

This material is also commonly referred to as N-para-benzene acetonitrile menthane carboxamide. *See e.g.* Research Disclosure RD 522003 (Givaudan), U.S. Patent Pub. Nos 2009/0311206 and 2009/0306152, both assigned to Beiersdorf, 2006/0276667, 2010/0086498, and U.S. Patent 7,414,152. Various methods to manufacture N-para-benzene acetonitrile menthane carboxamide have been disclosed, including in U.S. Patent Publ. 2006/027667, 2008/0300314, 2010/0040563, and 2010/0076080. N-para-benzene acetonitrile menthane carboxamide is commercially available from suppliers under CAS 852379-28-3, which can be supplied as a white powder with an assay of 94% to 100% and a melting point of 145°C at 760 mm Hg.

In these preferred embodiments, the skin engaging member comprises more than one thermally resilient sensate: a mixture of N-substituted menthanecarboxamide and menthane carboxylic acid-N-(4-methoxyphenyl)-amide, which it is believed provides improved cooling sensation properties, e.g. better instantaneous and lasting cooling and superior cooling overall. These two coolants may be combined at a ratio from about 25:1 to about 1:25 of N-substituted menthanecarboxamide to menthane carboxylic acid-N-(4-methoxyphenyl)-amide, alternatively from about 10:1 to about 1:10, alternatively from about 3:1 to about 1:3.

Without intending to be bound by theory, it is believed that N-substituted menthanecarboxamide triggers both TRPM8 and TRPA1 (Tingle/numb/burn) receptor, while menthane carboxylic acid-N-(4-methoxyphenyl)-amide triggers only the cooling receptor TRPM8, and L-menthol triggers TRPM8, TRPA1 and warming receptors TRPV1 & TRPV3. It is thus believed that the a system comprising one or both of the synthetic derivatives of cyclohexane described above, with the option of containing additional sensates makes it possible to achieve in-shave and long-last cooling benefits.

### c. Additional Sensates

In some embodiments, the skin engaging member further comprises one or more additional sensates other than the thermally resilient sensates disclosed above. For example, menthol is widely used as a cooling agent, but menthol can also produce other sensations including tingling, burning, prickling and stinging as well as a minty smell and bitter taste. Thus, it can be inferred that menthol acts on many different receptors, including cold, warm, pain and taste receptors. However, it is not readily discernible how to isolate which receptor activities would result in a specific sensation such as pleasant cooling without the undesirable sensations such as bitterness or irritation. Neither is it apparent how to control the activity of coolants or other sensory agents such that only the desired sensation is elicited from use of a particular sensory agent. As such, the present invention is focused on the addition of specific synthetic derivatives of cyclohexane (described above) to act as sensates to deliver cooling benefit to users during the hair removal process. Additional sensates can be used to further supplement the cooling feel.

A large number of coolant compounds of natural or synthetic origin are known. The most well-known compound is menthol, particularly 1-menthol, which is found naturally in peppermint oil, notably of *Mentha arvensis* L and *Mentha viridis* L. Of the isomers of menthol, the 1-isomer occurs most widely in nature and is typically what is referred by the name menthol having coolant properties. L-menthol has the characteristic peppermint odor, has a clean fresh taste and exerts a cooling sensation when applied to the skin and mucosal surfaces. Other isomers of menthol (neomenthol, isomenthol and neoisomenthol) have somewhat similar, but not identical odor and taste, i.e., some having disagreeable notes described as earthy, camphor, musty. The biggest difference among the isomers is in their cooling potency. L-menthol is reported to provide the most potent cooling, i.e., having the lowest cooling threshold (i.e., the concentration where the cooling effect could be clearly recognized) of about 800 ppb. At this level, there is no cooling effect for the other isomers. For example, d-neomenthol is reported to have a cooling threshold of about 25,000 ppb and 1-neomenthol about 3,000 ppb. [R. Emberger and R. Hopp, "Synthesis and Sensory Characterization of Menthol Enantiomers and Their Derivatives for the Use in Nature Identical Peppermint Oils," Specialty Chemicals (1987), 7(3), 193-201]. This study demonstrated the outstanding sensory properties of 1-menthol in terms or cooling and freshness and the influence of stereochemistry on the activity of these molecules.

Among synthetic coolants, many are derivatives of or are structurally related to menthol, i.e., containing the cyclohexane moiety, and derivatized with functional groups including carboxamide, ketal, ester, ether and alcohol. Examples include the p-menthanecarboxamide compounds such as N-ethyl-p-menthan-3-carboxamide, known commercially as "WS-3", and others in the series such as WS-5 (N-ethoxycarbonylmethyl-p-menthan-3-carboxamide), and WS-14 (N-tert-butyl-p-menthan-3-carboxamide). Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional examples of synthetic coolants include alcohol derivatives such as 3-(1-menthoxy)-propane-1,2-diol known as TK-10, isopulegol (under the tradename Coolact P) and ρ-menthane-3,8-diol (under the tradename Coolact 38D) all available from Takasago; menthone glycerol acetal known as MGA; menthyl esters such as menthyl acetate, menthyl acetoacetate, menthyl lactate known as Frescolat® supplied by Haarmann and Reimer, and monomenthyl succinate under the tradename Physcool from V. Mane. TK-10 is described in U.S. Pat. No. 4,459,425 to Amano et al. Other alcohol and ether derivatives of menthol are described e.g., in GB 1,315,626 and in U.S. Pat. Nos. 4,029,759; 5,608,119; and 6,956,139. WS-3 and other carboxamide cooling agents are described for example in U.S. Pat. Nos. 4,136,163; 4,150,052; 4,153,679; 4,157,384; 4,178,459 and 4,230,688. Additional N-substituted ρ-menthane carboxamides are described in WO 2005/049553A1 including N-(4-cyanomethylphenyl)-p-menthanecarboxamide, N-(4-sulfamoylphenyl)-p-menthanecarboxamide, N-(4-cyanophenyl)ₚ-menthanecarboxamide, N-(4-acetylphenyl)-p-menthanecarboxamide, N-(4-hydroxymethylphenyl)-p-menthanecarboxamide and N-(3-hydroxy-4-methoxyphenyl)-p-menthanecarboxamide. Other N-substituted ρ-menthane carboxamides include amino acid derivatives such as those disclosed in WO 2006/103401 and in U.S. Pat. Nos. 4,136,163; 4,178,459 and 7,189,760 such as N-((5-methyl-2-(1-methylethyl)cyclohexyl)carbonyl)glycine ethyl ester and N-((5-methyl-2-(1-methylethyl)cyclohexyl)carbonyl)alanine ethyl ester. Menthyl esters including those of amino acids such as glycine and alanine are disclosed e.g., in EP 310,299 and in U.S. Pat. Nos. 3,111,127; 3,917,613; 3,991,178; 5,5703,123; 5,725,865; 5,843,466; 6,365,215; 6,451,844; and 6,884,903. Ketal derivatives are described, e.g., in U.S. Pat. Nos. 5,266,592; 5,977,166 and 5,451,404. Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112. Reviews on the coolant activity of menthol and synthetic coolants include H. R. Watson, et al. J. Soc. Cosmet. Chem. (1978), 29, 185-200 and R. Eccles, J. Pharm. Pharmacol., (1994), 46, 618-630.

### II. Matrix Material

The skin engaging member further comprises a matrix material within which the thermally resilient sensate is present. The matrix material can be in various forms, as well as mixtures/combinations thereof:

### a. Solid Polymeric Matrix

In some embodiments, the matrix comprises a water soluble polymer selected from a polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof. In some embodiments, said water soluble polymer is selected from the group consisting of polyethylene oxide, polyethylene glycol, and mixtures thereof.

In some embodiments, the skin engaging member comprises any other ingredients commonly found in commercially available skin engaging members, such as those used on razor cartridges by Gillette, Schick or BIC. Non-limiting examples of such skin engaging members include those disclosed in U.S. 6301785, 6442839, 6298558, 6302785, and U.S Patent Pubs 2008/060201, and 2009/0223057. In some embodiments, the skin engaging member further comprises a skin engaging member ingredient selected from the group consisting of polyethylene oxide, polyvinyl pyrrolidone, polyacrylamide, hydroxypropyl cellulose, polyvinyl imidazoline, polyethylene glycol, poly vinyl alcohol, polyhydroxyethylmethacrylate, silicone copolymers, sucrose stearate, vitamin E, soaps, surfactants, panthenol, aloe, plasticizers, such as polyethylene glycol; beard softeners; additional lubricants, such as silicone oil, Teflon® polytetrafluoroethylene powders (manufactured by DuPont), and waxes; essential oils such as menthol, camphor, eugenol, eucalyptol, safrol and methyl salicylate; tackifiers such as Hercules Regalrez 1094 and 1126; non-volatile cooling agents, inclusion complexes of skin-soothing agents with cyclodextrins; fragrances; antipruritic/counterirritant materials; antimicrobial/keratolytic materials such as Resorcinol; anti-inflammatory agents such as Candilla wax and glycyrrhetinic acid; astringents such as zinc sulfate; surfactants such as pluronic and iconol materials; compatibilizers such as styrene-b-EO copolymers; mineral oil, polycaprolactone (PCL), and combinations thereof.

The water-soluble polymer will preferably comprise at least 50%, more preferably at least 60%, by weight of the skin engaging member, up to about 99%, or up to about 90% of the matrix. The more preferred water soluble polymers are the polyethylene oxides generally known as POLYOX (available from Union Carbide Corporation) or ALKOX (available from Meisei Chemical Works, Kyoto, Japan). These polyethylene oxides will preferably have molecular weights in unified atomic mass units, daltons, or g/mol (mol.wt.s) of about 100,000 to 6 million, most preferably about 300,000 to 5 million. The most preferred polyethylene oxide comprises a blend of about 40% to 80% of polyethylene oxide having an average mol.wt. of about 5 million (e.g. POLYOX COAGULANT) and about 60% to 20% of polyethylene oxide having an average mol.wt. of about 300,000 (e.g. POLYOX WSR-N-750). The polyethylene oxide blend may also advantageously contain up to about 10% by weight of a low mol.wt. (i.e. MW<10,000) polyethylene glycol such as PEG-100.

In some embodiments, the matrix further comprises from about 0.5% to about 50%, preferably from about 1% to about 20%, polycaprolactone (preferably mol.wt. of 30,000 to 60,000 daltons). See US 6,302,785.

In another embodiment, the skin engaging member may contain other conventional skin engaging member ingredients, such as low mol.wt. water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, antioxidants, preservatives, vitamin E, aloe, cooling agents, essential oils, beard softeners, astringents, medicinal agents, etc. Portions that contain a colorant can be designed to release the colorant (e.g., by leaching or abrasion), and thereby cause the strip to change color during shaving, preferably in response to wear of the colored portion, so as to provide an indication to the user that the skin engaging member and/or the razor cartridge has reached the end of its effective life or the end of its optimum performance. A portion may contain, for example, between about 0.1% and about 5.0% (preferably between about 0.5% and 3%) colorant by weight.

The matrix can further comprise a water-insoluble polymer in which the water-soluble polymer is dispersed. Preferably, at a level of from about 0% to about 50%, more preferably about 5% to about 40%, and most preferably about 15% to about 35% by weight of the skin engaging member of a water-insoluble polymer. Suitable water-insoluble polymers which can be used include polyethylene (PE), polypropylene, polystyrene (PS), butadiene-styrene copolymer (e.g. medium and high impact polystyrene), polyacetal, acrylonitrile-butadiene-styrene copolymer, ethylene vinyl acetate copolymer, polyurethane, and blends thereof such as polypropylene/polystyrene blend or polystyrene/impact polystyrene blend.

One preferred water-insoluble polymer is polystyrene, preferably a general purpose polystyrene, such as NOVA C2345A, or a high impact polystyrene (i.e. polystyrene-butadiene), such as BASF 495F KG21. The strip or any portion should contain a sufficient quantity of water-insoluble polymer to provide adequate mechanical strength, both during production and use.

### b. Emollients

In another embodiment, the matrix material comprises at least one emollient. In some embodiments the emollient is hydrophobic. In certain embodiments, the composition can consist essentially of one or more emollients which could form a fluid at 25 °C. Where the emollient is fluid form, the fluid is preferably contained within a skin engaging reservoir as disclosed below. In such embodiments, depending on the viscosity of the composition, varying orifice sizes can be used to control the dispensing of emollient during use.

The emollient is liquid, semi-solid and/or solid at room temp. The emollient may comprise one or more hydrocarbon emollients, a lipid, lipophilic skin care actives, or a mixture thereof. Suitable lipids include fatty acyls such as fatty acids, fatty alcohols, esters, triglycerides, fats, butters, and waxes; glycerolipids; glycerophospholipids; sphingolipids; sterol lipids; prenol lipids; saccharolipids; polyketides; lipophilic skin active agent emollients, and mixtures thereof.

Hydrocarbon emollients include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD™ from Croda) and Polydecene (such as Puresyn 2™ from Exxon Mobil).

Fatty alcohol or fatty acid emollients include saturated and unsaturated higher alcohols, especially C₁₂ - C₃₀ fatty alcohols and fatty acids, especially lauric, myristic, palmitic, stearic, arachidic or behenic. Ester emollients include esters of a C₁₂ - C₃₀ alcohol and mixtures thereof, especially isopropyl myristate, isopropyl isostearate and mixtures thereof. Triglyceride emollients include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol). Fat and butter emollients include coconut butter, shea butter and mixtures thereof. Wax emollients include paraffin wax, microcrystalline wax, candellila, ozokerite and mixtures thereof. Preferably, the emollient comprises paraffin wax. Advantageously, a hydrophobic phase comprises some wax because waxes may bestow further improved hardness and erodability to the solid moisturising composition. Preferably, the erodible, solid moisturizing composition comprises from about 2% to about 20% and more preferably from about 3% to about 15% wax by weight of the erodible, sold moisturizing composition.

Another class of suitable lipids include lipophilic skin active agent emollients which include oil soluble vitamins, such as vitamin E derivatives, including vitamin E acetate and tocopherol nicotinate; oil-soluble vitamin A derivatives, such as retinyl palmitate, lanolin, ceramides, sterols and sterol esters, salicylic acid, camphor, eucalyptol and essential oils.

In some embodiments, the matrix material comprises at least one emollient and a water insoluble structuring polymer. Examples of such compositions have been described as an erodable, solid moisturizing composition described in copending U.S. Patent Application Serial Nos. 13/0265556 entitled "HAIR REMOVAL DEVICE COMPRISING ERODABLE MOISTURIZER" and 13/026575 entitled "HAIR REMOVAL DEVICE COMPRISING AN ERODABLE MOISTURIZER", both to Stephens et al, filed Feb. 18, 2010.

As used herein, the term "solid" when used in relation to the erodable, solid moisturizing composition refers to compositions which are solid at 25°C. As used herein, the term "water-insoluble" when used in relation to the structuring polymer, means "very slightly soluble", according to the United States' Pharmacopeia (USP) definition in 31/NF 26 Vol. 2 General Notices, Page Xvii., or less than "very slightly soluble", which, using the USP definition, means that more than 1000 parts of solvent (water, in this case) are needed to dissolve 1 part of solute (the structuring polymer, in this case) at Standard Temperature and Pressure. As used herein, the term "soluble in" when describing the ability of the water-insoluble structuring polymer to dissolve in the hydrophobic phase means "soluble", according to the United States' Pharmacopeia definition in 31/NF 26 Vol. 2 General Notices, Page Xvii., or at least "soluble" using the USP definition, which means that less than 30 parts of solvent (the hydrophobic phase, in this case) are needed to dissolve 1 part of solute (the structuring polymer, in this case) at the melting point of the water-insoluble structuring polymer.

In some embodiments, the matrix with the emollient is an erodable, solid moisturizing composition having a Chatillon Hardness at 25°C of about 0.50kg to about 3.25kg, preferably about 0.75 kg to about 3.00kg, more preferably about 1.00kg to about 2.50kg, measured according to the protocol provided hereinbelow. It is believed that a skin conditioning composition having such Chatillon hardness provides beneficial rates of wear. The Chatillon Hardness Test is disclosed in U.S. Patent Application Serial No. 13/026556.

The water-insoluble structuring polymer when comprised within the erodable, solid moisturizing composition may be any water-insoluble structuring polymer which bestows appropriate wear properties to the erodable, solid moisturizing composition and is preferably a water-insoluble structuring polymer which may bestow a Chatillon Hardness in the above-defined ranges to the erodable, solid moisturizing composition. The structuring polymer is water-insoluble to assist miscibility with or solubility in the hydrophobic phase (at the melting point of the water-insoluble structuring polymer), which in turn may ensure a homogenous distribution of hydrophobic phase throughout the polymer and thus more even wear properties. In addition, the water soluble nature of the polymer may improve the durability of the polymer (and therefore also the erodible, solid moisturizing composition) versus more hydrophilic polymers which may solubilise and wash away during hair removal processes that employ water, such as wet shaving.

In some embodiments, the erodable, solid moisturizing composition comprises from about 2% to about 50%, preferably from about 3% to about 40%, more preferably about 4% to about 12% of water-insoluble structuring polymer by weight of the erodable, solid moisturizing composition. In some embodiments, the water-insoluble structuring polymer comprises a block copolymer. More advantageously, the block copolymer comprises a di-block copolymer, a tri-block copolymer, a multiblock copolymer, a radial block copolymer, a random block copolymer, or a mixture of these polymers. More advantageously still, the block copolymer comprises a tri-block copolymer.

Where the matrix material comprises the solid polymeric matrix, one or more emollients may also be included in the solid polymeric matrix.

### c. Soap Base

The matrix material may comprise a soap base, i.e. at least one soap or interrupted soap, e.g., a poured soap base or an extruded soap base. The basic component of the soap base can be a vegetable oil or tallow, saponified or neutralized to form the base, or can be a synthetic poured soap base. Super-fatted materials containing portions (e.g., greater than about 25 weight percent) of coconut acid or other fatty acids may also be used. In some embodiments, the matrix material includes a base comprising a vegetable oil or a tallow or the like, or a combination of the foregoing materials, which is saponified or neutralized. The saponification or neutralization of the vegetable oil or tallow results in the production of glycerol and salts of fatty acids to form the base. The matrix can include about 50 wt% to about 100 wt % saponified or neutralized base (e.g., about 75 wt% to about 100 wt% saponified or neutralized base), which may be opaque, translucent, or transparent. Exemplary salts of fatty acids that may be produced include sodium carboxylate salts having up to about 22 carbon atoms.

The soap base can be a synthetic soap base. In certain embodiments, the synthetic soap base includes a glycol (e.g., diproylene glycol, propylene glycol, tripropylene glycol, and/or methylpropane diol glycol), glycerin, fatty acid salts (e.g., sodium stearate and/or potassium stearate), C15-C25 alcohols (e.g., behenyl alcohol, stearyl alcohol, cetyl alcohol, and/or myristic alcohol), steareth (e.g., a steareth 21 such as, for example, Brij^{®}-721), stearic acid, microcrystalline wax (e.g., microcrystalline wax SP 16, SP 19, SP 16, SP 18, SP-1674, SP 16W, SP 60W, SP 89, Multiwax 180M, X-145, W-445, and/or W-835), one or more surfactants (e.g., Tegobetaine F-50, Lonzaine^{®}, the Mackam^{®} family of surfactants, the Mirataine^{®} family of surfactants, and sodium lauryl ether sulfate ("SLES") (e.g., 25% active SLES).

The soap base can, in certain embodiments, include from about 0.5% to about 30% glycol (e.g., from about 10% to about 25% glycol or from about 12% to about 15% glycol), from about 10% to about 40% glycerin (e.g., from about 18% to about 34% glycerin or from about 18% to about 24% glycerin), from about 20% to about 40% fatty acid salt (e.g., from about 25% to about 40% fatty acid salts (e.g., stearate) or from about 30% to about 35% fatty acid salt), from about 0.1% to about 10% stearic acid (e.g., from about 2 to about 5% stearic acid), from about 0.5% to about 10% microcrystalline wax (e.g., from about 0.5% to about 5% microcrystalline wax or from about 1% to about 3% microcrystalline wax), from about 1% to about 15% betaine (e.g., from about 2% to about 10% active betaine or from about 4% to about 9% active betaine), and from about 1 to about 20% active SLES (e.g., from about 1% to about 20% active SLES or from about 10% to about 15% active SLES), all based on the weight of the soap base. One exemplary poured soap base prior to addition of the thermally resilient sensate includes the following:

| | |
|---|---|
| Dipropylene glycol | 17.2% |
| Glycerin | 21.4% |
| Sodium stearate | 34.4% |
| Stearic acid (Pristerene^{®} 4980) | 3.7% |
| Microcrystalline wax SP 89 | 1.2% |
| Tegobetaine F-50 | 7.4% |
| SLES, 25% active | 14.7% |

In some embodiments, a combination of base and synthetic surfactants can be employed.

### d. Carrier

In some embodiments, the skin engaging member further comprises a carrier wherein the matrix, sensate and any other materials can be contained within the carrier and/or present on the carrier. The carrier can be in the form of a tray upon which the matrix material and encapsulated active are applied, or the carrier can form a retaining structure at least partially containing the matrix and encapsulated material. In some embodiments, the carrier forms a reservoir, for example from which shaving aid is dispensed to the skin with or without direct contact between the carrier and the skin, and such as the sheaths disclosed in U.S. Patent No. 6,298,558 and 7,581,318. Especially where the matrix material comprises an emollient in fluid form, but applicable generally, the carrier is preferably a sheath having one or more dispensing orifices to control the dispensing of one or more of the materials of the skin engaging member. When referring to the compositional make up of the skin engaging member, the weight percentages defined herein are determined based on the components of the skin engaging member disclosed herein but not the carrier, unless otherwise specified.

### e. Additional Actives in the Matrix

### i. Optional Cooling Agents

The matrix material may also comprise a neat non-volatile cooling agent or an inclusion complex of a skin-soothing agent with a cyclodextrin, preferably in amounts up to about 25%, most preferably about 10% to about 20%, by weight of the skin engaging member. "Neat" as used herein means that the additional actives are present outside the encapsulates and are dispersed within the remainder of the matrix material. By non-volatile cooling agent is meant an agent which has a physiological cooling effect on the skin and which is appreciably less volatile than menthol. Preferably, the nonvolatile cooling agent will be one which when subjected to thermogravimetric analysis (e.g. using a 951 Thermogravimetric Analyzer from Dupont with a 20°C temperature rise per minute) will retain at least about 50% of its initial weight at a temperature of 160°C, more preferably at least about 80% of its initial weight at a temperature of 160°C, and most preferably at least about 50% of its initial weight at a temperature of 175°C.

Suitable cooling agents which can be utilized include non-volatile menthol analogs such as menthyl lactate, menthyl ethoxyacetate, menthone glycerinacetal, 3-1-menthoxypropane-1,2-diol, ethyl 1-menthyl carbonate, (IS, 3S,4R)-p-menth-8-en-3-ol, menthyl pyrrolidone 25 carboxylate, N-substituted-p-menthane-3-carboxamides (as described in U.S. Pat. No. 4,136,163, which is incorporated herein by reference) including, for example, N-ethyl-p-menthane-3-carboxamide.

Suitable skin-soothing agents which can be utilized in the cyclodextrin inclusion complex include menthol, camphor, eugenol, eucalyptol, safrol, methyl salicylate, and the aforedescribed menthol analogs. Any suitable cyclodextrin may be utilized to form the inclusion complex including alphacyclodextrin, beta-cyclodextrin, gamma-cyclodextrin and modified cyclodextrins such as hydroxypropyl-beta-cyclodextrin, methyl-beta-cyclodextrin., and acetyl-betacyclodextrin. The preferred cyclodextrins are betacyclodextrin and gamma-cyclodextrin.

When the matrix material comprises a cyclodextrin inclusion complex, the matrix material may also advantageously comprise up to about 10%, preferably about 2% to about 7%, by weight of a displacing agent which displaces the skin-soothing agent from the inclusion complex upon contact with water, thereby enhancing the release of the skin-soothing agent from the skin engaging member material during use. The displacing agent is a material which is capable of forming a more stable complex with the cyclodextrin than the complex formed with the skin soothing agent and, thus, displaces the skin-soothing agent from the complex when the skin engaging member is contacted with water. Suitable displacing agents include surfactants, benzoic acids, and certain amines (e.g. urea). Further details with respect to the aforementioned cooling agents, cyclodextrin inclusion complexes and displacing agents may be found in U.S. Patent Nos. 5,653,971, and, 5,713,131.

Those of skill in the art will understand that one or more of the cooling agents listed in this section as Optional Cooling Agents can also be used as the cooling agent in a form encapsulated within either the nano-particle and/or the micro-particle. The matrix material can further comprise one or more other skin care actives in a neat form. Non-limiting examples of suitable other skin care actives include those disclosed throughout this specification.

### III. Encapsulated Actives

In some embodiments, the skin engaging member of the present invention further comprises at least one encapsulated active. The encapsulated active can be (e.g.) a thermally resilient sensate, an additional sensate, a perfume or another skin care active or composition. In some embodiments, the level of said at least one encapsulated active (including the weight of the capsule and encapsulated active) is from about 0.01 % to about 50 % by weight of said skin engaging member, alternatively from about 10 % to about 45 %, alternatively from about 15 % to about 35 %. The encapsulated actives can contain the same ingredients or different ingredients. The encapsulated actives can also include mixtures of ingredients.

Nonlimiting examples of encapsulation technology can be the nano and micro particles described in U.S. Patent No. 7,115,282. The nano-particles of the present invention are hydrophobic in nature. In some embodiments, the nano-particles have an average diameter in the range from about 0.01 micron to about 10 microns, or from about 0.05 microns to about 5 microns, or from about 0.1 microns to about 2 microns. This linear dimension for any individual particle represents the length of the longest straight line joining two points on the surface of the particle. In some embodiments, a portion of the nano-particles are encapsulated into one or more water-sensitive micro-particles. In some embodiments, the majority of the nano-particles present in the skin engaging member are encapsulated into said water-sensitive micro-particles. The micro-particles have an average particle size of from about 2.0 microns to about 100 microns, or from about 20 microns to about 100 microns.

The sensates of the present invention can be included as a neat ingredient (as a direct addition into the composition), in an encapsulate, or as a coating or separate layer. In some embodiments, one or more of the thermally resilient sensates can be present in both a neat form and in an encapsulate. In some embodiments, one of the thermally resilient sensates can be in a neat form and another sensate or thermally resilient sensate can be in an encapsulate. For example, in some embodiments, the N-substituted menthanecarboxamide can be in a neat form and the menthane carboxylic acid-N-(4-methoxyphenyl)-amide can be in the capsule. In another embodiment, the N-substituted menthanecarboxamide can be in encapsulated and the menthane carboxylic acid-N-(4-methoxyphenyl)-amide can be in neat form. Further, in another embodiment, an additional sensate or optional cooling agent (such as disclosed herein) can be present in a neat form along with one or both of the thermally resilient sensates of the present invention. For example, any of the above thermally resilient sensates can be used along with L-menthol, menthyl lacrate, or any other commonly used cooling agent, all as neat product, or with one or more cooling agents or sensates in the capsule.

In some embodiments the level of active or actives in the encapsulated active ranges from about 20% to about 90%, preferably from about 30% to about 75% by weight of the nano-particles. In some embodiments the level of the active or actives in the encapsulated active ranges from about 10% to about 60%, or from about 30% to about 50% by weight of the micro-particles. Lower levels of the encapsulated active can also be used, e.g. as low as 10%, or as low as 5%, or as low as 1%.

In some embodiments, encapsulated active comprises more than one cooling agent, for example L-menthol + Menthyl lactate (Frescolat ML); L-menthol + Menthone Glycerine Acetal (Frescolat MGA); or L-menthol + Coolact 10, or peppermint oil. In yet another embodiment, the encapsulated active comprises at least one cooling agent and a fragrance, a mineral oil, or a combination thereof. In another embodiment, the cooling agent comprises a mixture of menthol and menthyl lactate, such as described in WO 2007115593 (commercially available as Fresocolat Plus), or the eutectic mixture of menthol and menthyl lactate in a ratio of weight in the range of 1:4 to 4:1, as described in U.S. 6,897,195.

### IV. Additional Skin Care Active Ingredients

Various skin care actives ("actives") which are commonly used for topical application can be included in the skin engaging member as a neat product and / or in an encapsulate.

Non-limiting examples of suitable cooling agents include: L-menthol; p-menthane-3,8-diol; Isopulegol; Menthoxypropane-1,2,-diol; Curcumin; Menthyl Lactate (such as Frescolat ML by Symrise); Gingerol; Icilin; Tea Tree Oil; Methyl Salicylate; Camphor; Peppermint Oil; N-Ethyl-p-menthane-3-carboxamide; Ethyl 3-(p-menthane-3-carboxamido)acetate; 2-Isopropyl-N,2,3-trimethylbutyramide; Menthone glycerol ketal, Menthone Glyerine Acetal; Coolact 10; and mixtures thereof. These and other cooling agents are known and described in various publications, such as U.S. Patent No. 2008/0300314A1, U.S. Patent No. 5,451,404 and 7,482,373. In yet another embodiment, the cooling agent comprises one or more of the cooling agents previously described for use in various shave aids. *See e.g.,* U.S. Patent Nos. 5,095,619; 5,713,131; 5,095,619; 5,653,971; 6,298,558; 6,944,952; and 6,295,733.

Other actives suitable for cosmetic and dermatological use can be used herein. Non-limiting examples of suitable actives include one or more of: Bis-abolol and ginger extract, a surfactant derived from olive oil such as Olivem 450® and Olivem 460®, Lauryl p-Cresol Ketoxime, 4-(1-Phenylethyl)1,3-benzenediol, Lupin (Lupinus albus) oil & wheat (Triticum vulgare) germ oil unsaponifiables, Hydrolyzed lupin protein, Extract of L-lysine and L-arginine peptides, Oil soluble vitamin C, Evodia rutaecarpa fruit extract, Zinc pidolate and zinc PCA, Alpha-linoleic acid, p-thymol, and combinations thereof; at least one additional skin and/or hair care active selected from the group consisting of sugar amines, vitamin B₃, retinoids, hydroquinone, peptides, farnesol, phytosterol, dialkanoyl hydroxyproline, hexamidine, salicylic acid, N-acyl amino acid compounds, sunscreen actives, water soluble vitamins, oil soluble vitamins, hesperedin, mustard seed extract, glycyrrhizic acid, glycyrrhetinic acid, carnosine, Butylated Hydroxytoluene (BHT) and Butylated Hydroxyanisole (BHA), menthyl anthranilate, cetyl pyridinium chloride, tetrahydrocurmin, vanillin or its derivatives, ergothioneine, melanostatine, sterol esters, idebenone, dehydroacetic acid, Licohalcone A, creatine, creatinine, feverfew extract, yeast extract (e.g., Pitera®), beta glucans, alpha glucans, diethylhexyl syringylidene malonate, erythritol, p-cymen-7-ol, benzyl phenylacetate, 4-(4-methoxyphenyl)butan-2-one, ethoxyquin, tannic acid, gallic acid, octadecenedioic acid, p-cymen-5-ol, methyl sulfonyl methane, an avenathramide compound, fatty acids (especially poly-unsaturated fatty acids), antifungal agents, thiol compounds (e.g., N-acetyl cysteine, glutathione, thioglycolate), other vitamins (vitamin B 12), beta-carotene, ubiquinone, amino acids, their salts, their derivatives, their precursors, and/or combinations thereof; and a dermatologically acceptable carrier. These and other potentially suitable actives are described in greater detail in U.S. Patent Publication No. 2008/0069784.

Additional actives that can be used include those commercially available under the following tradenames: Signaline S, Jojoba Oil, Ceramidone, Net DG, Pal-GHK (Paltenex), Rhodysterol, Vital ET, and combinations thereof.

In another embodiment, the active can be a methyl naphthalenyl ketone. The methyl naphthalenyl ketone can be a 1-(1,2,3,4,5,6,7,8-octahydro-2,3,8,8-tetramethyl-2naphthalenyl)-ethan-1-one molecule or an isomer or derivative thereof. Commercially available as Iso-E-Super from IFF of New York. Other sensates can also be used, including those which have ability to up-regulate the TRPM8 receptor, which has been described as the cool menthol receptor. Non-limiting examples of suitable TRPM8 regulators include: p-menthane-3,8-diol; Isopulegol; Menthoxypropane-1,2,-diol; Curcumin; Menthyl Lactate; Gingerol; Icilin; Menthol; Tea Tree Oil; Methyl Salicylate; Camphor; Peppermint Oil; N-Ethyl-p-menthane-3-carboxamide; Ethyl 3-(p-menthane-3-carboxamido)acetate; 2-Isopropyl-N,2,3-trimethylbutyramide; Menthone glycerol ketal, and mixtures thereof.

The active ingredient can also be one or more skin care actives suitable for topical use. The CTFA Cosmetic Ingredient Handbook, Second Edition (1992) describes a wide variety of nonlimiting cosmetic and pharmaceutical ingredients commonly used in the skin care industry, which are suitable for use in the compositions of the present invention. Examples of these ingredient classes include: abrasives, absorbents, aesthetic components such as fragrances, pigments, colorings/colorants, essential oils, skin sensates, astringents, etc. (e.g., clove oil, camphor, eucalyptus oil, eugenol, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, fatty alcohols and fatty acids, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, skin-conditioning agents, skin soothing and/or healing agents and derivatives, skin treating agents, thickeners, and vitamins and derivatives thereof. Additional non-limiting examples of additional suitable skin treatment actives are included in U.S. 2003/0082219 in Section I (i.e. hexamidine, zinc oxide, and niacinamide); U.S. 5,665,339 at Section D (i.e. coolants, skin conditioning agents, sunscreens and pigments, and medicaments); and US 2005/0019356 (i.e. desquamation actives, anti-acne actives, chelators, flavonoids, and antimicrobial and antifungal actives). It should be noted, however, that many materials may provide more than one benefit, or operate via more than one mode of action. Therefore, classifications herein are made for the sake of convenience and are not intended to limit the active to that particular application or applications listed.

### V. Hair Removal Head

According to some embodiments of the invention, a hair removal device is provided, which generally comprises a hair removal head and a handle or grip portion, upon which the hair removal head is mounted. The hair removal device can be manual or power driven and can be used for wet and/or dry application. The hair removal head is a razor cartridge where the device is a shaving razor. The hair removal head may be replaceable and/or pivotally connected to a cartridge connecting structure and in turn or independently (e.g. permanently fixed) to a handle. In some embodiments, the cartridge connecting structure includes at least one arm to releasably engage the hair removal head.

The hair removal head typically comprises one or more elongated edges usually positioned between a first and second end, said one or more elongated edges comprising a tip extending towards said first end. Where the hair removal head is a razor cartridge the one or more elongated edges can include blades. For example, U.S. Patent 7,168,173 generally describes a Fusion® razor that is commercially available from The Gillette Company and which includes a razor cartridge with multiple blades. Additionally, the razor cartridge may include a guard as well as a skin engaging member. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the present skin engaging member can be used with any currently marketed system or disposable razor, including those having 2, 3, 4 or 5 blades. In such a case, the hair removal device is a razor, the hair removal head is a razor cartridge and the one or more elongated edges are blades. Another example of a hair removal device is a scraping tool for use with a hair removal composition, i.e. a depilatory.

In some embodiments, said at least one skin engaging member is located on the portion of the cartridge that contacts skin during the hair removal process, forward and/or aft of the blades. A feature "forward" of the one or more elongated edges, for example, is positioned so that the surface to be treated with by the hair removal device encounters the feature before it encounters the elongated edges. A feature "aft" of the elongated edge is positioned so that the surface to be treated by the hair removal device encounters the feature after it encounters the elongated edges. Where more than one skin engaging member is provided on the hair removal device, they can be the same (identical) or different, in terms of physical shape/structure and/or chemical composition, and one or more of them may comprise the sensate.

In some particular embodiments, a plurality (e.g. 2, a first and second) of skin engaging members may be provided on the hair removal head, with the first skin engaging member comprising menthane carboxylic acid-N-(4-methoxyphenyl)-amide according to Formula A or preferably Formula B and the second skin engaging member comprising an N-substituted menthanecarboxamide according to Formula I or preferably Formula II (even more preferably with Formula B in the first skin engaging member). These skin engaging members may be placed collectively (for example adjacent to one another) ahead of or behind the elongated edges (e.g. blades on a razor cartridge), including side by side, or separately with one ahead of the elongated edges and the other behind.

In some embodiments, the cartridge comprises a guard comprising at least one elongated flexible protrusion to engage a user's skin. The at least one flexible protrusion may comprise flexible fins generally parallel to said one or more elongated edges. Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower surface). In some embodiments, said skin engaging member is positioned on the cartridge aft of the guard and forward of said elongated edge. In another embodiment, the skin engaging member is positioned on the cartridge forward of the guard. This embodiment can be particularly useful to deliver the skin engaging member prior to contact with the guard.

### VI. Method of Making

Skin engaging members of the present invention may be fabricated by any appropriate method, including injection molding, pressing, impregnation, spray-coating, calendaring and extrusion, or combinations of such steps.. All of the components of the strip, including the thermally resilient sensates can be blended prior to molding or extrusion. For best results, it is preferred that the components are dry.

The blended components may be extruded through a Haake System 90, ¾ inch diameter extruder with a barrel pressure of about 1000-2000 psi, a rotor speed of about 10 to 50 rpm, and a temperature of about 150°-185°C and a die temperature of about 170°-185°C. Alternatively, a 1¼ inch single screw extruder may be employed with a processing temperature of 175°-200°C, preferably 185°-190°C, a screw speed of 20 to 50 rpm, preferably 25 to 35 rpm, and an extrusion pressure of 1800 to 5000 psi, preferably 2000 to 3500 psi. The extruded strip is air cooled to about 25°C. To injection mold the strips it is preferred to first extrude the powder blend into pellets. This can be done on a 1¼ or 1½ inch single screw extruder at a temperature of 120°-180°C, preferably 140°-150°C, with a screw speed of 20 to 100 rpm, preferably 45 to 70 rpm. The pellets are then molded in either a single material molding or multi-material molding machine, which may be single cavity or multicavity, optionally equipped with a hot-runner system. The process temperature can be from 165° to 250°C, preferably from 180° to 225°C. The injection pressure should be sufficient to fill the part completely without flashing. Depending on the cavity size, configuration and quantity, the injection pressure can range from 300 to 2500 psi. The cycle time is dependent on the same parameters and can range from 3 to 30 seconds, with the optimum generally being about 6 to 15 seconds.

### VII. Details on Figures

Referring to Figs. 1 and 2, the razor cartridge (an example of a hair removal head) 14 includes housing 16, which carries three blades 18, a finned elastomeric guard 20, and a skin engaging member 22 located on a skin-engaging portion (in this case the cap) of the cartridge. Skin engaging member 22 is shown having two layers, the first layer can be the matrix and encapsulated active of the present invention, and the second layer can be a conventional shave aid, or vice versa. The skin engaging member is preferably locked in (via adhesive, a fitment, or melt bonding) an opening in the rear of the cartridge. Skin engaging member 32, shown in Fig. 3, is similar to skin engaging member 22, except that skin engaging member 32 has a homogeneous composition throughout and a uniform, slightly curved to flat upper surface. This type of skin engaging member may also be fabricated in a wedge-shaped cross-section or any other desired shape. The skin engaging member may also be constructed in two or more layers, such as a sandwich or a sheath/core construction.

The present invention may also include a method of use of a skin engaging shaving aid member to provide a cooling, tingling, refreshing, or otherwise topically noticeable sensation or feeling to a user by applying a skin engaging shaving aid member in accordance with at least one embodiment of the present invention onto a users' skin. This can be done as part of a process or method of shaving.

### VIII. Examples

Examples 1 - 4 can be made according to the below tables with the following method: ingredients are blended and mixed with other ingredients in a tumbler to make a homogeneous powder. The obtained powder is then single extruded into lubrastrips at 160-180°C and 100-200 bar pressure.

| **Ingredients** | **Example 1** | **Example 2** |
|---|---|---|
| | wt% | wt% |
| Dow Polyox Coagulant (PEO) | 39.73 | 36.73 |
| Dow Polyox N-750 w/4% Vitamin E | 26.44 | 24.44 |
| Polystyrene 731G HIPS with Acrowax | 15.15 | 15.15 |
| Dow Carbowax 4600PEG | 4.75 | 4.75 |
| Dow Tone Polymer P-767 | 4.70 | 4.70 |
| Ciba-Geigy B215 Irganox Antioxidant | 0.24 | 0.24 |
| Aloe | 0.19 | 0.19 |
| Colorant | 3.80 | 3.80 |
| Givaudan G180 | 0.00 | 5.00 |
| Symrise SC1 (Frescolat MMC) | 5.00 | 5.00 |

| **Ingredients** | **Example 3** | **Example 4** |
|---|---|---|
| | wt% | wt% |
| Dow Polyox Coagulant (PEO) | 20.16 | 16.16 |
| Dow Polyox N-750 | 39.01 | 39.01 |
| ECM High Impact Polystyrene Pulverised 5410 | 19.15 | 19.15 |
| Dow Carbowax 4600PEG | 4.75 | 4.75 |
| Solvay PCL Tone Polymer Capa 6506S | 4.70 | 4.70 |
| Ciba-Geigy B215 Irganox Antioxidant | 0.24 | 0.24 |
| Protameen Freeze dried Aloe | 0.19 | 0.19 |
| ECM CHIPS490FF Green Concentrate (Colorant) | 3.80 | 3.80 |
| Symrise SC1 (Frescolat MMC) | 8.00 | 12.00 |

Examples 3 and 4 were made and tested by ten male shavers who shave at least four times per week and who are cooling sensitive, meaning they were pre-screened by shaving a razor product with cooling lubrastrip against a razor product with non-cooling lubrastrip, using a split face protocol and indicating that they could perceive the cooling sensation and thus discriminate the two razor products. Each person has shaved three razor products with lubrastrip as the only variant. A can of Gillette Series Sensitive Skin Shave Gel was used for each shave to minimize the shave prep variation effect on cooling sensitivity detection. The shaving context for each person was kept as close as possible through the entire shave test, for example the only variation between razors was the lubrastrip and the same shaving preparation (Gillette Series Sensitive Skin Shave Gel) was used for every experiment. The shaving order of products was randomized in order to cancel possible product interference. Each razor product was used for five normal shaves. The ten shavers scored their perceived cooling during and after shaving on a scale from 0 to 10 (where 0 indicates no cooling sensation perceived). These scores were averaged and the results appear in the table below:

| | Example 3 | Example 4 |
|---|---|---|
| Cooling intensity during shaving | 2.74 | 1.52 |
| Cooling intensity after shaving | 4.04 | 3.18 |

The results show that the coolants are able to withstand the extrusion process and provide shaver-noticeable cooling benefit both during and after shaving. Importantly, with these test subjects, the cooling intensity was shown to increase after shaving compared to during shaving. This was surprising and unexpected as the additional of many other coolants in general into such shaving aids was not originally considered to be able to provide meaningful noticeable impacts on many users.

The following comparative example, containing 5% menthol, was also produced using the method above, and tested by four male shavers who shave at least four times per week and who are cooling sensitive as above. The shavers reported they were unable to detect any cooling effect.

| **Ingredients** | **Comparative Example 1** |
|---|---|
| | wt% |
| Dow Polyox Coagulant (PEO) | 34.85 |
| Dow Polyox N-750 w/4% Vitamin E | 23.19 |
| Polystyrene Nova 5410 HIPS | 13.29 |
| Dow Carbowax 4600PEG | 4.17 |
| Solvay PCL Tone Polymer Capa 6506S | 4.13 |
| Ciba-Geigy B215 Irganox Antioxidant | 0.21 |
| Aloe | 0.17 |
| Colorant | 3.33 |
| Salvona MultiSal 160 L-Menthol (30% menthol load) | 16.66 |

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein. Similarly, it should be understood that each feature of the each specified embodiment of the invention may be independently applied to each other specified embodiment, as if all such combinations were expressly written herein, unless these combinations are specifically excluded or the relevant features are innately incompatible (e.g. the features are directly contradictory).

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Except as otherwise noted, the articles "a," "an," and "the" mean "one or more."

## Claims

1. A hair removal device comprising:
a. a hair removal head (14);
b. one or more elongated edges (18) positioned on said cartridge; and
c. the skin engaging member positioned on said cartridge, said skin engaging member (22) comprising:
a. a matrix comprising at least one of: a water soluble polymer, selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof, an emollient, a soap base, and a mixture thereof;
b. a thermally resilient sensate comprising a menthane carboxylic acid-N-(4-methoxyphenyl)-amide of formula:

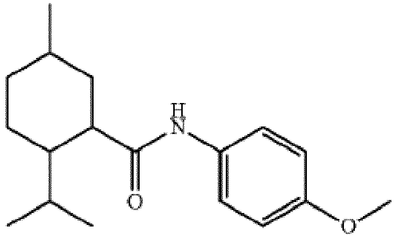

2. A hair removal device of claim 1, wherein said menthane carboxylic acid-N-(4-methoxyphenyl)-amide has the formula:

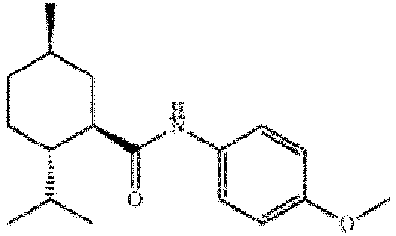

3. A hair removal device of claim 1 or 2, wherein said thermally resilient sensate further comprises a N-substituted menthanecarboxamide having the formula: 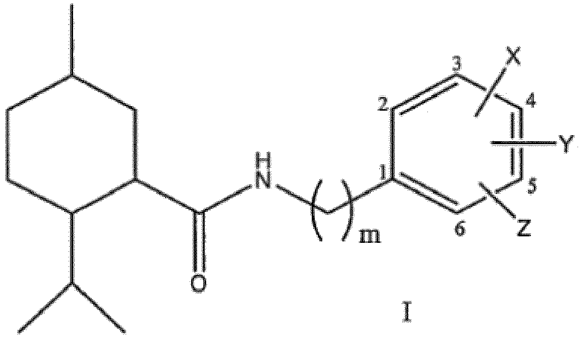 in which m is 0 or 1, Y and Z are selected independently from the group consisting of H, OH, C1-C4 straight or branched alkyl, or, a C1-C4 straight or branched alkoxy, X is (CH2)n-R, where n is 0 or 1 and R is a group with non-bonding electrons, selected from halogens, OH, OMe, NO2, CN, Ac, SO2NH2, CHO, CO2H and C1-C4 alkyl carboxylates, subject to the provisos that: (a) when Y and Z are H, X is not F, OH, MeO or NO2 in the 4-position and is not OH in the 2 or 6-position, and (b) when Y or Z is H then X, Y and Z are such that (i) the groups in the 3- and 4-positions are not both OMe, (ii) the groups in the 4- and 5-positions are not both OMe, (iii) the groups in 3- and 5-positions are not OMe if the group in the 4-position is OH, and (iv) the groups in the 3- and 5-positions are not OH if the group in the 4- position is methyl.

4. A hair removal device of claim 3, wherein X is in the 4-position, preferably wherein X is in the 4-position and Y and Z are H, OH, Me or OMe.

5. A hair removal device of any of claims 3 to 4, wherein said thermally resilient sensate comprises N-[4-(cyanomethyl)phenyl]-(1R,2S,5R)-2-isopropyl-5-methylcyclohexanecarboxamide of formula:

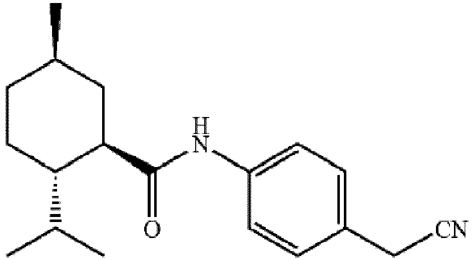

6. A hair removal device of any preceding claim, further comprising an additional cooling agent selected from the group consisting of: L-menthol; p-menthane-3,8-diol; Isopulegol; Menthoxypropane-1,2,-diol; Curcumin; Menthyl Lactate; Gingerol; Icilin; Tea Tree Oil; Methyl Salicylate; Camphor; Peppermint Oil; N-Ethyl-p-menthane-3-carboxamide; Ethyl 3-(p-menthane-3-carboxamido)acetate; 2-Isopropyl-N,2,3-trimethylbutyramide; Menthone glycerol ketal, Menthone Glyerine Acetal; and mixtures thereof, preferably wherein said optional cooling agent is a mixture of menthol and menthyl lactate, more preferably wherein the mixture of menthol and menthyl lactate is in a ratio of from 1:4 to 4:1 by weight.

7. A hair removal device of any preceding claim, wherein the level of thermally resilient sensate is from 0.01% to 25%.

8. A hair removal device of any preceding claim, wherein said water soluble polymer is present at a level of from about 50% to about 90% by weight of said solid polymeric matrix.

9. A hair removal device of any preceding claim, wherein said matrix further comprises a water insoluble polymer, preferably comprising at least one of: polyethylene, polypropylene, polystyrene, high impact polystyrene, butadiene styrene copolymer, polyacetal, acrylonitrile-butadiene styrene copolymer, ethylene vinyl acetate copolymer, and mixtures thereof. wherein said water insoluble polymer is preferably present at a level of from 5% to 40%, preferably from 15% to 35% by weight of the skin engaging member.

10. A hair removal device according to claim 1, further comprising a second skin engaging member positioned on said cartridge, said second skin engaging member being according to any of claims 1-9, or comprising:
a. a matrix comprising at least one of: a water soluble polymer, selected from polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof, an emollient, a soap base, and a mixture thereof;
b. thermally resilient sensate further comprises a N-substituted menthanecarboxamide having the formula: 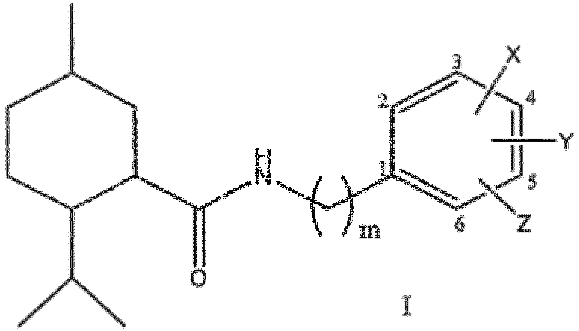 in which m is 0 or 1, Y and Z are selected independently from the group consisting of H, OH, C1-C4 straight or branched alkyl, or, a C1-C4 straight or branched alkoxy, X is (CH2)n-R, where n is 0 or 1 and R is a group with non-bonding electrons, selected from halogens, OH, OMe, NO2, CN, Ac, SO2NH2, CHO, CO2H and C1-C4 alkyl carboxylates, subject to the provisos that: (a) when Y and Z are H, X is not F, OH, MeO or NO2 in the 4-position and is not OH in the 2 or 6-position, and (b) when Y or Z is H then X, Y and Z are such that (i) the groups in the 3- and 4-positions are not both OMe, (ii) the groups in the 4- and 5-positions are not both OMe, (iii) the groups in 3- and 5-positions are not OMe if the group in the 4-position is OH, and (iv) the groups in the 3- and 5-positions are not OH if the group in the 4- position is methyl.

11. A hair removal device according to claims 1 or 10, wherein the hair removal device is a razor, the hair removal head is a razor cartridge and the one or more elongated edges are blades.

12. A method of making a hair removal device according to any preceding claim comprising the steps of:
a. providing a polymeric matrix and the thermally resilient sensate to form a feed;
b. preferably heating said feed to a temperature of from about 120°C to about 200°C, and
c. forming a skin engaging shaving aid member.

13. The method of claim 12, wherein said step of forming is selected from the group consisting of: extruding said feed into said skin engaging shaving aid member; molding said feed said feed into said skin engaging shaving aid member; injection molding said feed into said skin engaging shaving aid member; and a combination thereof.

## Patentansprüche

1. Haarentfernungsvorrichtung, umfassend:
a. ein Haarentfernungskopfstück (14);
b. eine oder mehrere langgestreckte Kanten (18), die auf der Rasierklingeneinheit angeordnet sind; und
c. das auf der Rasierklingeneinheit angeordnete Hauteingriffselement, wobei das Hauteingriffselement (22) Folgendes umfasst:
a. eine Matrix, umfassend zumindest eines von: einem wasserlöslichen Polymer, ausgewählt aus Polyethylenoxid, Polyvinylpyrrolidon, Polyacrylamid, Polyhydroxymethacrylat, Polyvinylimidazolin, Polyethylenglykol, Polyvinylalkohol, Polyhydroxyethymethacrylat, Silikonpolymeren und Mischungen davon, einem Weichmacher, einer Seifenbasis und einer Mischung davon;
b. ein thermisch nachgiebiges Sensat, umfassend ein Menthancarbonsäure-N-(4-methoxyphenyl)-amid mit der Formel:

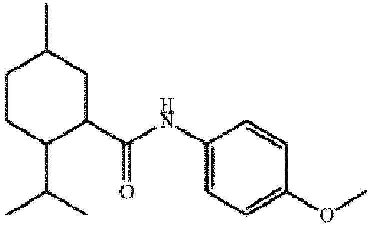

2. Haarentfernungsvorrichtung nach Anspruch 1, wobei das Menthancarbonsäure-N-(4-methoxyphenyl)-amid folgende Formel aufweist:

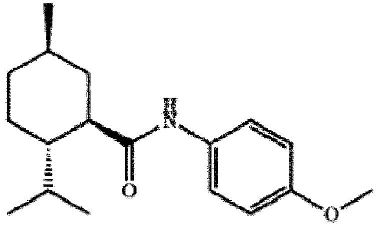

3. Haarentfernungsvorrichtung nach Anspruch 1 oder 2, wobei das thermisch robuste Sensat ferner ein N-substituiertes Menthancarboxamid umfasst mit der Formel: 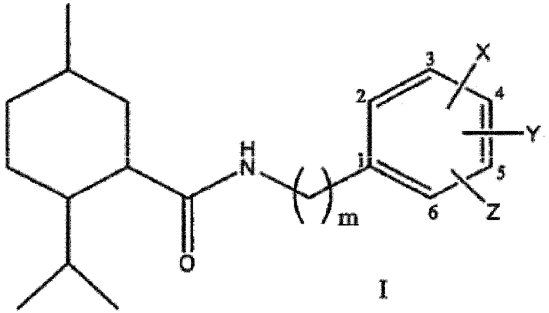 wobei m 0 oder 1 ist, Y und Z unabhängig ausgewählt sind aus der Gruppe, bestehend aus H, OH, C1-C4 geradkettigem oder verzweigtem Alkyl oder einem geradkettigen oder verzweigten C1-C4-Alkoxy, X für (CH2)n-R steht, wobei n 0 oder 1 ist, und R für eine Gruppe mit nicht-bindenden Elektronen steht, ausgewählt aus Halogenen, OH-, OMe-, NO2-, CN-, Ac-, SO2NH2-, CHO-, CO2H- und C1-C4-Alkylcarboxylaten, gemäß der Maßgabe, dass: (a) wenn Y und Z H sind, X in der 4-Stellung nicht F, OH, MeO oder NO2 ist und in der 2- oder 6-Stellung nicht OH ist, und (b) wenn Y oder Z H ist, dann sind X, Y und Z so gewählt, dass (i) die Gruppen in den 3- und 4-Stellungen nicht beide OMe sind, (ii) die Gruppen in den 4- und 5-Stellungen nicht beide OMe sind, (iii) die Gruppen in 3- und 5-Stellungen nicht OMe sind, wenn die Gruppe in der 4-Stellung OH ist, und (iv) die Gruppen in den 3- und 5-Stellungen nicht OH sind, wenn die Gruppe in der 4-Stellung Methyl ist.

4. Haarentfernungsvorrichtung nach Anspruch 3, wobei X in 4-Stellung steht, vorzugsweise wobei X in 4-Stellung steht und Y und Z für H, OH, Me oder OMe stehen.

5. Haarentfernungsvorrichtung nach einem der Ansprüche 3 bis 4, wobei das thermisch robuste Sensat ein N-[4-(Cyanomethyl)phenyl]-(1R,2S,5R)-2-isopropyl-5-methylcyclohexancarboxamid umfasst mit der Formel:

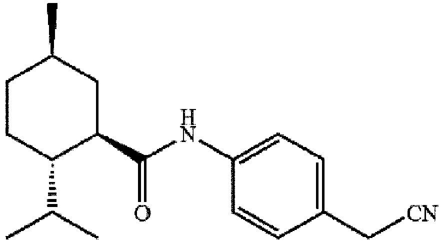

6. Haarentfernungsvorrichtung nach einem der vorstehenden Ansprüche, ferner umfassend ein zusätzliches Kühlmittel, ausgewählt aus der Gruppe bestehend aus: L-Menthol; P-Menthan-3,8-diol; Isopulegol; Menthoxypropan-1,2,-diol; Kurkumin; Menthyllactat; Gingerol; Icilin; Teebaumöl; Methylsalicylat; Kampfer; Pfefferminzöl; N-Ethyl-p-menthan-3-carboxamid; Ethyl-3-(p-menthan-3-carboxamido)acetat; 2-Isopropyl-N,2,3-trimethylbutyramid; Menthonglycerinketal, Menthonglycerinacetal; und Mischungen davon, wobei das optionale Kühlmittel vorzugsweise ein Gemisch aus Menthol und Menthyllactat ist, wobei das Gemisch aus Menthol und Menthyllactat mehr bevorzugt im Gew.-Verhältnis von 1:4 bis 4:1 steht.

7. Haarentfernungsvorrichtung nach einem der vorstehenden Ansprüche, wobei der Gehalt an thermisch robustem Sensat von 0,01 % bis 25 % beträgt.

8. Haarentfernungsvorrichtung nach einem der vorstehenden Ansprüche, wobei das Niveau an wasserlöslichem Polymer etwa 50 Gew.-% bis etwa 90 Gew.-% der Festpolymermatrix beträgt.

9. Haarentfernungsvorrichtung nach einem der vorstehenden Ansprüche, wobei die Matrix ferner ein wasserunlösliches Polymer umfasst, das vorzugsweise wenigstens eines der Folgenden umfasst: Polyethylen, Polypropylen, Polystyrol, hochschlagfestes Polystyrol, Butadien-Styrolcopolymer, Polyacetal, Acrylonitril-Butadien-Styrolcopolymer, Ethylenvinyl-Acetatcopolymer, und Mischungen davon, wobei das wasserunlösliche Polymer vorzugsweise in einem Anteil von 5 % bis 40 %, vorzugsweise von 15 Gew.-% bis 35 Gew.-% des Hauteingriffselements vorhanden ist.

10. Haarentfernungsvorrichtung nach Anspruch 1, ferner ein zweites Hauteingriffselement umfassend, das auf der Rasierklingeneinheit angeordnet ist, wobei das zweite Hauteingriffselement einem der Ansprüche 1-9 entspricht oder Folgendes umfasst:
a. eine Matrix, umfassend zumindest eines von: einem wasserlöslichen Polymer, ausgewählt aus Polyethylenoxid, Polyvinylpyrrolidon, Polyacrylamid, Polyhydroxymethacrylat, Polyvinylimidazolin, Polyethylenglykol, Polyvinylalkohol, Polyhydroxyethymethacrylat, Silikonpolymeren und Mischungen davon, einem Weichmacher, einer Seifenbasis und einer Mischung davon;
b. ein thermisch nachgiebiges Sensat, ferner umfassend ein N-substituiertes Menthancarboxamid mit der Formel: 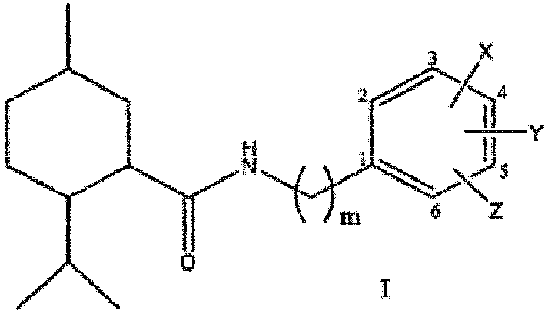 wobei m 0 oder 1 ist, Y und Z unabhängig ausgewählt sind aus der Gruppe, bestehend aus H, OH, C1-C4 geradkettigem oder verzweigtem Alkyl oder einem geradkettigen oder verzweigten C1-C4-Alkoxy, X für (CH2)n-R steht, wobei n 0 oder 1 ist, und R für eine Gruppe mit nicht-bindenden Elektronen steht, ausgewählt aus Halogenen, OH-, OMe-, NO2-, CN-, Ac-, SO2NH2-, CHO-, CO2H- und C1-C4-Alkylcarboxylaten, gemäß der Maßgabe, dass: (a) wenn Y und Z H sind, X in der 4-Stellung nicht F, OH, MeO oder NO2 ist und in der 2- oder 6-Stellung nicht OH ist, und (b) wenn Y oder Z H ist, dann sind X, Y und Z so gewählt, dass (i) die Gruppen in den 3- und 4-Stellungen nicht beide OMe sind, (ii) die Gruppen in den 4- und 5-Stellungen nicht beide OMe sind, (iii) die Gruppen in 3- und 5-Stellungen nicht OMe sind, wenn die Gruppe in der 4-Stellung OH ist, und (iv) die Gruppen in den 3- und 5-Stellungen nicht OH sind, wenn die Gruppe in der 4-Stellung Methyl ist.

11. Haarentfernungsvorrichtung nach Ansprüchen 1 oder 10, wobei die Haarentfernungsvorrichtung ein Rasierer und das Haarentfernungskopfstück eine Rasierklingeneinheit ist, und die eine oder mehreren verlängerten Kanten Klingen sind.

12. Verfahren zur Herstellung einer Haarentfernungsvorrichtung nach einem der vorstehenden Ansprüche, umfassend die Schritte:
a. Zurverfügungstellen einer Polymermatrix und des thermisch nachgiebigen Sensates, um einen Zulauf zu bilden;
b. vorzugsweise Erwärmen des Zulaufs auf eine Temperatur von etwa 120 °C bis 200 °C, und
c. Ausbilden eines hautberührenden Rasierhilfsmittel-Elements.

13. Verfahren nach Anspruch 12, wobei der Schritt des Ausbildens ausgewählt wird aus der Gruppe, die besteht aus: Extrudieren des Zulaufs in das hautberührende Rasierhilfsmittel-Element; Gießen des Zulaufs in das hautberührende Rasierhilfsmittel-Element; Spritzgießen des Zulaufs in das hautberührende Rasierhilfsmittel-Element; und einer Kombination davon.

## Revendications

1. Dispositif d'épilation comprenant :
a. une tête d'épilation (14) ;
b. un ou plusieurs bords allongés (18) positionnés sur ladite cartouche ; et
c. l'élément venant en contact avec la peau positionné sur ladite cartouche, ledit élément de contact avec la peau (22) comprenant :
a. une matrice comprenant au moins l'un des éléments suivants : un polymère soluble dans l'eau choisi parmi l'oxyde de polyéthylène, la polyvinylpyrrolidone, le polyacrylamide, le polyhydroxyméthacrylate, la polyvinylimidazoline, le polyéthylène glycol, l'alcool polyvinylique, le polyhydroxyéthyméthacrylate, les polymères de silicone et des mélanges de ceux-ci, un émollient, une base de savon et un mélange de ceux-ci ;
b. un agent sensoriel thermiquement résilient comprenant un acide carboxylique menthane N-(4-méthoxyphényl)-amide de formule :

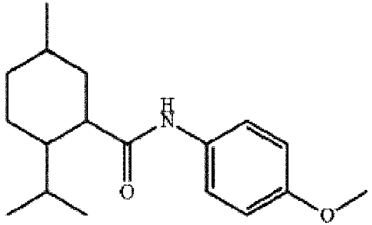

2. Dispositif d'épilation selon la revendication 1, dans lequel la formule dudit acide carboxylique menthane N-(4-méthoxyphényl)-amide est :

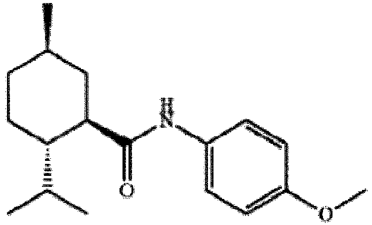

3. Dispositif d'épilation selon la revendication 1 ou 2, dans lequel ledit agent sensoriel thermiquement résilient comprend en outre un menthanecarboxamide N-substitué de formule : 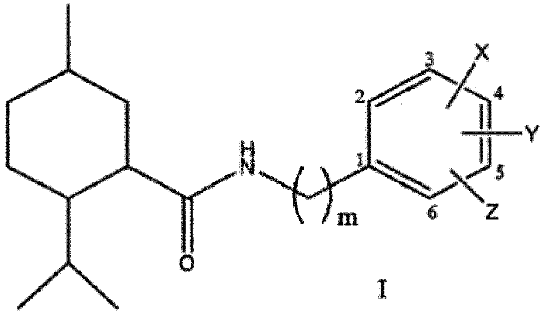 dans lequel m est égal à 0 ou 1, Y et Z sont choisis indépendamment dans le groupe constitué de H, OH, alkyle linéaire ou ramifié en C1-C4 ou un alcoxy linéaire ou ramifié en C1-C4, X représente (CH2) n-R, où n est égal à 0 ou 1 et R est un groupe avec des électrons non liés, choisi parmi les halogènes, OH, OMe, NO2, CN, Ac, SO2NH2, CHO, CO2H et alkylcarboxylates en C1-C4, sous réserve que : (a) lorsque Y et Z sont H, X n'est pas F, OH, MeO ou NO2 en position 4 et n'est pas OH en position 2 ou 6, et (b) lorsque Y ou Z est H, X, Y et Z sont tels que (i) les groupes dans les positions 3 et 4 ne sont pas tous deux OMe, (ii) les groupes dans les positions 4 et 5 ne sont pas tous deux OMe, (iii) les groupes dans les positions 3 et 5 ne sont pas OMe si le groupe en position 4 est OH, et (iv) les groupes dans les positions 3 et 5 ne sont pas OH si le groupe dans la position 4 est le méthyle.

4. Dispositif d'épilation selon la revendication 3, dans lequel X est dans la position 4, de préférence dans lequel X est dans la position 4 et Y et Z sont H, OH, Me ou OMe.

5. Dispositif d'épilation selon l'une quelconque des revendications 3 à 4, dans lequel ledit agent sensoriel thermiquement résilient comprend du N-[4-(cyanométhyl)phényl]-(1R,2S,5R)-2-isopropyl-5-méthylcyclohexanecarboxamide de formule :

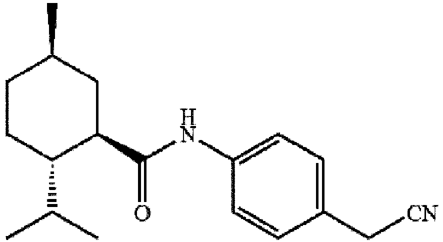

6. Dispositif d'épilation selon l'une quelconque des revendications précédentes, comprenant, en outre, un agent de refroidissement supplémentaire choisi dans le groupe constitué de : L-menthol ; p-menthane-3,8-diol ; isopulégol ; menthoxypropane-1,2,-diol ; curcumine ; lactate de menthyle ; gingérol ; iciline ; huile de théier ; salicylate de méthyle ; camphre ; essence de menthe poivrée ; N-éthyl-p-menthane-3-carboxamide ; 3-(p-menthane-3-carboxamido)acétate d'éthyle ; 2-isopropyl-N,2,3-triméthylbutyramide ; menthone glycérol cétal, menthone glycérine acétal ; et des mélanges de ceux-ci, de préférence dans lequel ledit agent de refroidissement facultatif est un mélange de menthol et de lactate de menthyle, plus préférablement dans lequel le mélange de menthol et de lactate de menthyle se trouve dans un rapport de 1:4 à 4:1 en poids.

7. Dispositif d'épilation selon l'une quelconque des revendications précédentes, dans lequel la proportion de l'agent sensoriel thermiquement résilient est comprise entre 0,01 % et 25 %.

8. Dispositif d'épilation selon l'une quelconque des revendications précédentes, dans lequel ledit polymère soluble dans l'eau est présent dans une proportion comprise entre environ 50 % et environ 90 % en poids de ladite matrice polymère solide.

9. Dispositif d'épilation selon l'une quelconque des revendications précédentes, dans lequel ladite matrice comprend en outre un polymère insoluble dans l'eau, comprenant de préférence au moins l'un des éléments suivants : polyéthylène, polypropylène, polystyrène, polystyrène choc, copolymère de butadiène styrène, polyacétal, copolymère acrylonitrile-butadiène styrène, copolymère éthylène-acétate de vinyle et des mélanges de ceux-ci, dans lequel ledit polymère insoluble dans l'eau est de préférence présent dans une proportion comprise entre 5 % et 40 %, de préférence entre 15 % et 35 % en poids de l'élément venant en contact avec la peau.

10. Dispositif d'épilation selon la revendication 1, comprenant en outre un deuxième élément venant en contact avec la peau positionné sur ladite cartouche, ledit deuxième élément de contact avec la peau étant selon l'une quelconque des revendications 1 à 9, ou comprenant :
a. une matrice comprenant au moins l'un des éléments suivants : un polymère soluble dans l'eau choisi parmi l'oxyde de polyéthylène, la polyvinylpyrrolidone, le polyacrylamide, le polyhydroxyméthacrylate, la polyvinylimidazoline, le polyéthylène glycol, l'alcool polyvinylique, le polyhydroxyéthyméthacrylate, les polymères de silicone et des mélanges de ceux-ci, un émollient, une base de savon et un mélange de ceux-ci ;
b. un agent sensoriel thermiquement résilient comprenant en outre un menthanecarboxamide N-substitué de formule : 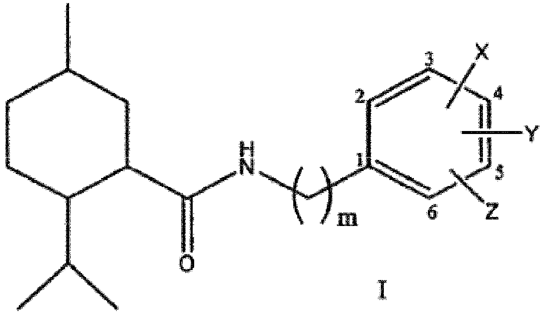 dans lequel m est égal à 0 ou 1, Y et Z sont choisis indépendamment dans le groupe constitué de H, OH, alkyle linéaire ou ramifié en C1-C4 ou un alcoxy linéaire ou ramifié en C1-C4, X représente (CH2) n-R, où n est égal à 0 ou 1 et R est un groupe avec des électrons non liés, choisi parmi les halogènes, OH, OMe, NO2, CN, Ac, SO2NH2, CHO, CO2H et alkylcarboxylates en C1-C4, sous réserve que : (a) lorsque Y et Z sont H, X n'est pas F, OH, MeO ou NO2 en position 4 et n'est pas OH en position 2 ou 6, et (b) lorsque Y ou Z est H, X, Y et Z sont tels que (i) les groupes dans les positions 3 et 4 ne sont pas tous deux OMe, (ii) les groupes dans les positions 4 et 5 ne sont pas tous deux OMe, (iii) les groupes dans les positions 3 et 5 ne sont pas OMe si le groupe en position 4 est OH, et (iv) les groupes dans les positions 3 et 5 ne sont pas OH si le groupe dans la position 4 est le méthyle.

11. Dispositif d'épilation selon la revendication 1 ou 10, dans lequel le dispositif d'épilation est un rasoir, la tête d'épilation est une cartouche de rasoir et le ou les bords allongés sont des lames.

12. Procédé de fabrication d'un dispositif d'épilation selon l'une quelconque des revendications précédentes, comprenant les étapes consistant à :
a. fournir une matrice polymère et l'agent sensoriel thermiquement résilient afin de former un flux d'alimentation ;
b. de préférence chauffer ledit flux d'alimentation à une température comprise entre 120 °C environ et 200 °C environ, et
c. former un élément d'aide au rasage venant en contact avec la peau.

13. Procédé selon la revendication 12, dans lequel ladite étape de formation est choisie dans le groupe constitué de : extrusion dudit flux d'alimentation en ledit élément d'aide au rasage venant en contact avec la peau ; moulage dudit flux d'alimentation dans ledit élément d'aide au rasage venant en contact avec la peau ; moulage par injection dudit flux d'alimentation dans ledit élément d'aide au rasage venant en contact avec la peau ; et une combinaison de celles-ci.
